(19) Europäisches Patentamt
European Patent Office
Office européen des brevets

(11) Publication number: **0 506 200 A1**

(12) **EUROPEAN PATENT APPLICATION**

(21) Application number: **92200866.9**

(22) Date of filing: **26.03.92**

(51) Int. Cl.[5]: **C07C 17/20**, C07C 17/00, C07C 25/13

(30) Priority: **27.03.91 US 676016**

(43) Date of publication of application: **30.09.92 Bulletin 92/40**

(84) Designated Contracting States: **CH DE ES FR GB IT LI NL**

(71) Applicant: **DOWELANCO**
**9002 Purdue Road**
**Indianapolis, Indiana 46268-3030(US)**

(72) Inventor: **Campbell, Kent D.**
**3939 Bayview Circle**
**Concord, California 94520(US)**
Inventor: **Fung, Alexander P.**
**1941 Spring Lake Drive**
**Martinex, California 94553(US)**
Inventor: **Wilson, Charles A.**
**Marina Boulevard, P.O. Box 1355**
**Pittsburg, California 94565(US)**

(74) Representative: **Smulders, Theodorus A.H.J., Ir. et al**
**Vereenigde Octrooibureaux Nieuwe Parklaan 97**
**NL-2587 BN 's-Gravenhage(NL)**

(54) **A process for the preparation of o-chlorofluorobenzene.**

(57) o-Chlorofluorobenzene is prepared from 1,2,3-trichlorobenzene by partial fluorine exchange to a mixture of 2,6-dichlorofluorobenzene and 2,3-dichlorofluorobenzene followed by selective reduction to a mixture of fluorobenzene, o-chlorofluorobenzene and 2,3-dichlorofluorobenzene. Each of the components of this latter mixture can be separated by a relatively easy distillation, and each has value as an intermediate in the manufacture of other valuable organic chemicals.

EP 0 506 200 A1

The present invention concerns a process for preparing o-chlorofluorobenzene from 1,2,3-trichlorobenzene. The process is characterized by the steps of partial fluorine exchange, selective reduction and separation.

o-Chlorofluorobenzene is useful in preparing valuable intermediates in the manufacture of a variety of chemical products including dyes, pharmaceuticals and agricultural chemicals. U.S. Patent 4,940,821, for example, describes the use of o-chlorofluorobenzene in the preparation of o-fluorophenol.

Conventional methods of preparing halofluoroaromatic compounds are based primarily on diazotization routes involving a number of steps. In U.S. Patent 4,476,320, for example, halofluoroaromatic compounds were prepared by (a) diazotizing the corresponding haloaromatic amine compound to the diazonium salt, and (b) decomposing the salt to the desired product:

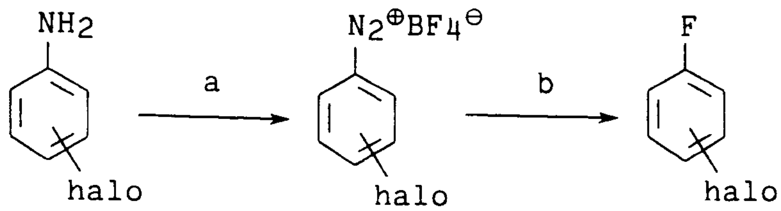


A similar scheme was employed in U.S. Patent 3,950,444 and in Japanese Kokai Tokkyo Koho 59-67,232. Alternatively, halofluorobenzenes have been prepared by the halogenation of fluorobenzenes, but the fluorobenzene starting materials are themselves usually prepared by the above-mentioned diazonium chemistry. Primarily of laboratory interest, several techniques for the fluorination of halobenzenes have been disclosed. These procedures include fluorination with fluorine atoms (*J.Fluorine Chem.*, **3**, 397 (1973)), with acetyl hypofluorite (*J.Org.Chem.*, **51**, 1886 (1986)) and with $AgF_2$ (*J.Org.Chem.*, **45**, 3597 (1980)). Recently, chlorofluorobenzenes have been prepared by the partial fluorine exchange of dichlorobenzenes with KF or CsF (U.S. Patent 4,937,397) and by the reduction of dihalofluorobenzenes (U.S. Patent 4,918,251).

Shiley *et al.*, in *J.Fluorine Chem.*, **2**, 19 (1972), disclose the fluorination of 1,2,3-trichlorobenzene with KF in dimethyl sulfone. Such fluorine exchanges typically produce a mixture of all possible products:

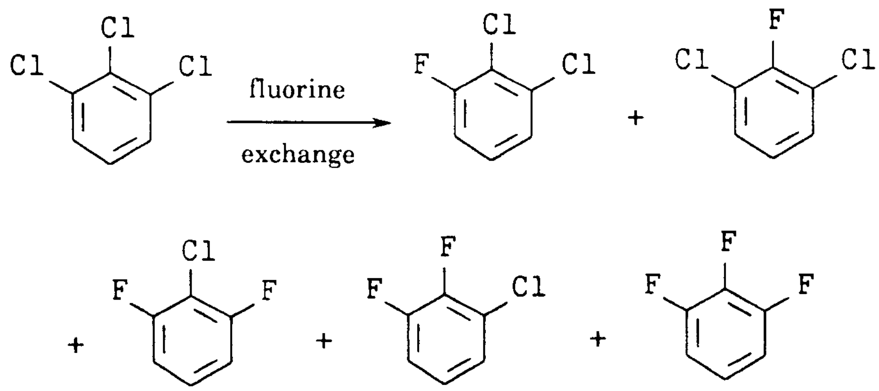


The difluorochlorobenzenes, for example, are useful intermediates in the preparation of certain agricultural chemicals. If they are to be economically manufactured by such a process, however, it is necessary to continuously recover and recycle the dichlorofluorobenzenes. In order to avoid recycle and to make the overall scheme more economical, it is desirable to have means of converting the dichlorofluorobenzene isomers into useful products.

The present invention concerns a process for preparing o-chlorofluorobenzene and 2,3-dichlorofluorobenzene from 1,2,3-trichlorobenzene by the following reaction scheme:

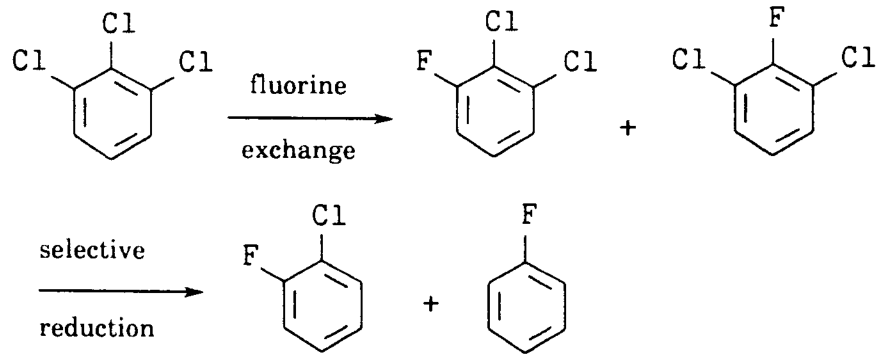


According to the present invention, the process is characterized by the following steps:

(a) exchanging one chlorine from 1,2,3-trichlorobenzene with fluorine to give a mixture of 2,6-dichlorofluorobenzene and 2,3-dichlorofluorobenzene;

(b) selectively reducing chlorine from the 2,6-dichlorofluorobenzene in admixture with 2,3-dichlorofluorobenzene by contacting the mixture with a hydrogen source in the presence of a palladium catalyst in an inert organic solvent at 50 to 70 °C to give a mixture of fluorobenzene, *o*-chlorofluorobenzene and 2,3-dichlorofluorobenzene; and

(c) separating the components from the mixture and from each other.

Fluorobenzene is itself a useful and valuable synthetic intermediate as is 2,3-dichlorofluorobenzene. By following the partial fluorine exchange with the selective reduction, three valuable intermediates can be prepared from a previously unutilized portion of a process stream ultimately derived from a readily available and relatively inexpensive starting material, i.e., 1,2,3-trichlorobenzene.

The partial fluorine exchange is typically accomplished by the action of fluoride ion on 1,2,3-trichlorobenzene. The conversion of 1,2,3-trichlorobenzene to a mixture containing 2,6-dichlorofluorobenzene and 2,3-dichlorofluorobenzene is a stepwise process which produces a mixture of products.

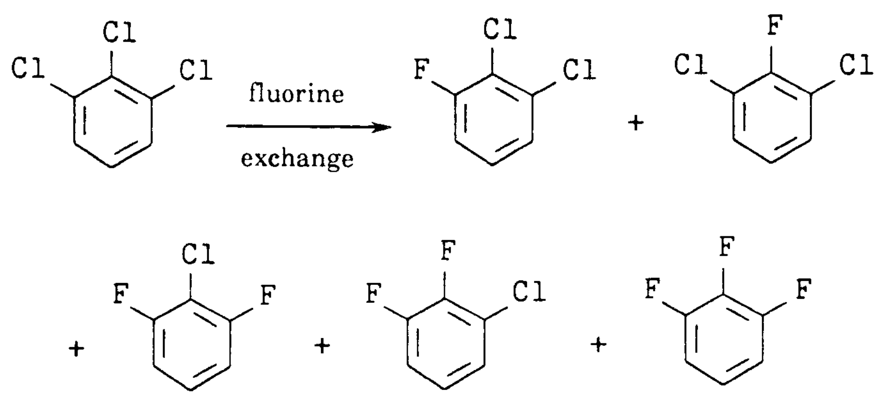


Those components of the mixture with different degrees of fluorine content, e.g., one fluorine as opposed to two fluorines, are usually easily separated from one another by distillation. Those components having the same fluorine content, on the other hand, oftentimes have very similar boiling points and are difficult to separate by distillation. Although the dichlorofluorobenzenes can be easily separated from the other components of the mixture by distillation, they cannot be so easily separated from one another.

The fluorine exchange reaction is effectively conducted by contacting the 1,2,3-trichlorobenzene with an effective amount of KF or CsF under substantially anhydrous conditions in a suitable polar aprotic solvent at a temperature so that fluorine exchange readily occurs.

KF and CsF, which are the usual fluorinating agents employed, are commercially available compounds. Substantially anhydrous and finely-divided KF or CsF are preferred. Amorphous or spray-dried forms are particularly preferred. Substantially anhydrous KF and CsF can be prepared, for example, by drying *in vacuo* at 140-250 °C for several hours.

Suitable polar aprotic diluents include N-methyl pyrrolidinone (NMP), N-cyclohexyl pyrrolidinone (NCHP), 1,3-dimethyl-2-imidazolidinone (DMI) and 1,3-dimethyl-3,4,5,6-tetrahydro-2-(1H)pyrimidone (DMTHP).

The fluorine exchange is conducted under substantially anhydrous conditions at elevated temperatures of from 170 to 290 ° C. Preferred temperature ranges are from 200 to 250 ° C when CsF is used, and from 250 to 290 ° C when KF is used.

Pressures of from atmospheric to greater than atmospheric are typically employed. For CsF, which is more reactive than KF, it is often convenient to operate at atmospheric pressure. For KF, which is less expensive than but also less reactive than CsF, it is preferred to operate at the autogenous pressure generated by the diluent, starting material and product in a sealed reactor at the preferred reaction temperatures of 250 to 290 ° C. Such pressures typically range from slightly above atmospheric to about 3.55 MPa [500 pounds per square inch gauge (psig)] and depend upon the volume of the reactor. Optionally, the reaction can be run under pressure in a suitably designed reactor equipped with a distillation column so the product can be removed as formed.

Water is detrimental to the reaction and substantially anhydrous reaction conditions are preferred. By substantially anhydrous is meant that the reaction medium contains less than 500 parts per million (ppm) water. Preferably the reaction medium contains less than 150 ppm water. Substantially anhydrous conditions may be achieved employing standard drying techniques. For example, a typical laboratory reactor can be dried by distilling the polar aprotic solvent under a vacuum before addition of the reactants. Optionally, a small amount (5-10 percent by weight of the polar aprotic solvent) of a non-polar solvent such as an aromatic hydrocarbon (toluene, xylene, etc.) may be added to the polar aprotic solvent to aid in the removal of water by azeotropic distillation. Residual water in the reaction mixture is also often removed by azeotropic distillation.

The amount of polar aprotic solvent is not critical, but it is advantageous to employ enough solvent to keep the starting material in solution at reaction temperatures, generally from 2 to 25 parts by weight of the solvent per part by weight of the 1,2,3-trichlorobenzene. The reaction consumes the reactants in the ratio of one mole of fluorinating agent per mole of chlorine atoms exchanged. Since a mixture of dichlorofluorobenzenes is desired, about 1 molar equivalent of KF or CsF per mole of starting material is consumed. Often it is beneficial to use more than a 10 percent excess of fluorinating agent. This is particularly so if the product, which has a lower boiling point than the starting material, is removed by distillation as it is formed.

In carrying out the fluorine exchange, usually the solvent and fluorinating agent are added to an appropriate reaction vessel, and the medium is dried by distilling a small portion of the solvent. The starting material is then added to the reaction vessel, and the reaction mixture is heated to a temperature high enough to maintain a satisfactory reaction rate. The product may be recovered from the reaction mixture after completion of the reaction by extraction or by flash distillation. The desired dichlorofluorobenzene fraction of the reaction mixture can conveniently be isolated by distillation.

In practice, however, the dichlorofluorobenzene fraction may not be the product for which the exchange reaction has been designed and optimized. For example, if the difluorochlorobenzenes are the preferred product, the reaction will generally be conducted with at least 2 molar equivalents of KF or CsF per mole of starting material. Since very little trifluorobenzene is formed in the exchange, an excess of fluorinating agent may be beneficial and from 3.0 to 4.0 moles of KF or CsF can be employed per mole of 1,2,3-trichlorobenzene. In any case, the optimization of a particular product mix is well within the skill of a practitioner in the art.

In the reduction step, the mixture of 2,6-dichlorofluorobenzene and 2,3-dichlorofluorobenzene is contacted with a hydrogen source in the presence of a palladium catalyst. During the course of the reaction, the chloro groups are selectively removed. Furthermore, the chloro substituents of the 2,6-dichlorofluorobenzene can be preferentially reduced in the presence of the corresponding 2,3-dichloro isomer.

The selective reduction is fairly specific to palladium catalysts, and palladium on carbon has generally been found to be more effective than palladium dispersed on other supports. Thus, the most preferred catalysts range from 0.5 to 10 weight percent palladium on carbon. Generally, from 0.01 to 0.20 parts of palladium are employed per part of dichlorofluorobenzene; from 0.01 to 0.10 parts are preferred.

The reduction can be conducted using hydrogen gas as the hydrogen source. The hydrogen gas can be continuously sparged into the reaction mixture at atmospheric pressure or the reaction mixture can be pressurized with hydrogen gas in a sealed reactor. With hydrogen gas, however, it is sometimes difficult to control the extent of reduction.

Formate salts are often convenient hydrogen sources. By the term "formate salts" is meant alkali metal formates, such as sodium formate and potassium formate, ammonium formate and trialkylammonium formates wherein the alkyl groups are straight-chained alkyl groups of 1 to 4 carbons, such as triethylammonium formate. The trialkylammonium formates, being relatively non-hygroscopic, easily prepared and quite soluble in most organic solvents, are among the preferred formate salts.

The trialkylammonium formates can be prepared by stirring an excess of trialkylamine with formic acid

in toluene. Removal of the solvent and excess amine by distillation leaves the trialkylammonium formate as a residue which can then be diluted with the desired solvent to give a reagent solution of known concentration. As an alternative to preforming the trialkylammonium formate solution, this reagent can be prepared *in situ* by the addition of a stoichiometric excess of trialkylamine to 96 percent formic acid in conjunction with the palladium catalyst during the reduction in a fashion similar to that described by Cortese *et al.*, *J.Org.Chem.*, **42**, 3491 (1977).

The reduction is typically performed using near stoichiometric amounts of reagents. Thus, from 0.9 to 1.1 equivalents of hydrogen source are usually employed for each equivalent of substrate to be reduced. However, for more sluggish reactions up to a 2-fold excess of the hydrogen source can be tolerated without forfeiture of selectivity.

The reduction is generally conducted in an organic solvent that is inert to the reaction conditions. Aliphatic nitriles, aliphatic alcohols, aromatic hydrocarbons and the polar aprotic solvents recommended for the partial fluorine exchange are particularly preferred. These solvents may be used with or without small amounts of added water, e.g., from 0.01 to 0.20 parts of water per part of solvent. With respect to the nitriles, acetonitrile is most preferred. With respect to the alcohols, $C_2$ to $C_4$ alcohols and glycols are preferred. 2-Propanol and ethylene glycol are particularly preferred for those reactions using an alkali metal formate as the hydrogen source. With respect to aromatic hydrocarbons, toluene is preferred. Aromatic hydrocarbons are acceptable solvents for the trialkylammonium formates but are unacceptable for the alkali metal formates, which are essentially insoluble in this class of solvents.

The reduction is generally carried out at a temperature from ambient to 150 °C, preferably from 50 to 70 °C. Operating pressures are not critical and may vary from atmospheric pressure to 4.93 MPa [700 pounds per square inch gauge (psig)]. Pressures from atmospheric to 1.48 MPa [200 psig] are preferred.

Since the reduction of the aromatic chlorines produces hydrogen chloride, at least one equivalent of an HCl acceptor should be added for each chlorine reduced to buffer the system. Such buffers can include, for example, alkali metal carbonates and acetates or organic amines such as pyridine, alkylamines or alkanolamines.

In a typical reduction reaction the mixture of 2,6-dichlorofluorobenzene and 2,3-dichlorofluorobenzene is introduced into a reactor along with a solvent, a palladium on carbon catalyst and the hydrogen source, e.g., a formate salt. The mixture is stirred at 50 to 70 °C until the chloro substituent has been removed from the 2,6-dichlorofluorobenzene. After cooling and venting, the reaction mixture can be isolated by conventional procedures such as filtration and extraction. The fluorobenzene, *o*-chlorofluorobenzene and 2,3-dichlorofluorobenzene can be separated from the reaction mixture and each other by distillation.

The following examples illustrate the invention. All melting points and boiling points are uncorrected.

Example 1 Partial Fluorine Exchange Reaction: KF

A 600 milliliter (mL) Hastelloy C Pressure reactor was charged with 58 grams (g) (1.0 mol) of KF, 350 mL of N-methyl pyrrolidinone (NMP) and 45.4 g (0.25 mol) of 1,2,3-trichlorobenzene. The reactor was sealed and pressure tested. The reaction mixture was stirred at 270 °C for 24 hours (hr). After cooling and venting the reactor, the reaction mixture was analyzed by gas chromatography (GC) using an internal standard method. The analysis indicated the following composition: 1,2,3-trichlorobenzene (TCB; 10 percent); dichlorofluorobenzenes ($Cl_2FB$; 48 percent); difluorochlorobenzenes ($ClF_2B$; 22 percent); and 1,2,3-trifluorobenzene (TFB; <1 percent). The aromatics were flash distilled from the mixture and the distillate was redistilled on a spinning band column. The dichlorofluorobenzene fraction had a boiling point of 172-178 °C.

Example 2

A series of KF exchanges was conducted following the general procedures of Example 1. The experimental conditions and results of these experiments are summarized in Table 1.

Table 1

KF Exchange on 1,2,3-Trichlorobenzene

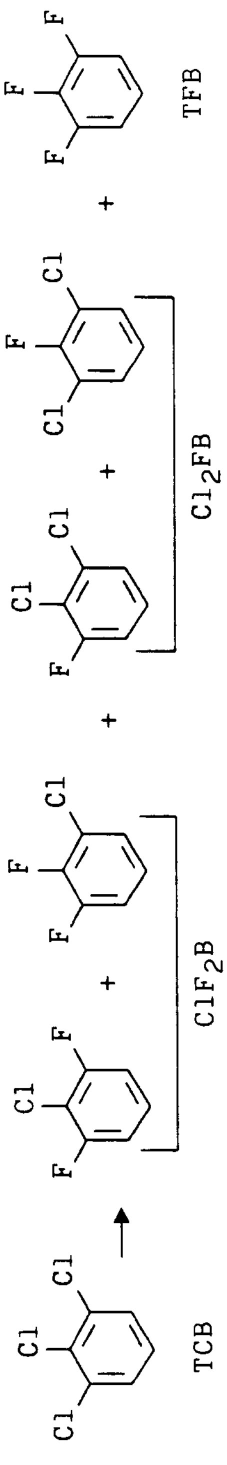

TCB

$ClF_2B$

$Cl_2FB$

TFB

| | mol | | | | mol% | | | |
|---|---|---|---|---|---|---|---|---|
| Solvent | TCB | KF | T(°C) | T(hr) | TCB | $ClF_2B$ | $Cl_2FB$ | TFB |
| DMTHP | 0.25 | 1.0 | 270 | 24 | 4 | 32 | 40 | <1 |
| DMTHP | 0.25 | 1.0 | 280 | 12 | 4-8 | 28-38 | 40-46 | ~1 |
| DMTHP | 0.25 | 1.0 | 290 | 12 | 1 | 40 | 22 | ~1 |
| DMI | 0.25 | 1.0 | 270 | 24 | 4 | 34 | 42 | <1 |
| DMI | 0.25 | 1.0 | 280 | 18 | 2 | 42 | 34 | 2 |
| DMI | 0.25 | 1.0 | 280 | 30 | 1 | 34 | 18 | 2 |
| NMP | 0.25 | 1.0 | 280 | 18 | 6 | 27 | 40 | ~1 |
| NMP | 0.25 | 1.0 | 290 | 12 | 4 | 30 | 38 | 1.5 |
| DMI | 0.50 | 1.0 | 290 | 12 | 3-4 | 35-38 | 39-40 | 1 |

DMTHP - 1,3-dimethyl-3,4,5,6-tetrahydro-2-(1H)pyrimidone
DMI - 1,3-dimethyl-2-imidazolidinone
NMP - N-methyl pyrrolidinone

Example 3 Partial Fluorine Exchange Reaction: CsF

A 600 mL Hastelloy C pressure reactor was charged with 152 g (1.0 mol) of CsF, 350 mL of 1,3-dimethyl-2-imidazolidinone (DMI) and 90.8 g (0.5 mol) of 1,2,3-trichlorobenzene. The reactor was sealed and pressure tested. The reaction mixture was stirred at 250°C for 12 hr. After cooling and venting the reactor, the reaction mixture was analyzed by GC using an internal standard method. TCB (<1 percent); $Cl_2FB$ (32 percent); $CF_2B$ (62 percent); and TFB (3 percent).

Example 4

To a 2000 mL 4-necked nickel flask equipped with a mechanical stirrer and a 15 tray vacuum-jacketed Oldershaw column was added 953 mL of DMI. Approximately 5 g of solvent was removed by distillation at 19.95 kPa (150 tor) to dry the system. The vacuum was released with nitrogen and 271 g (1.5 mol) of 1,2,3-trichlorobenzene and 608 g (4.0 mol) of finely ground CsF were added. The reaction mixture was stirred at 220 ° C and reaction progress was monitored GC. After completion of the reaction (<2.5 percent starting material), 222 g of volatiles comprised of the following components were isolated as mixtures by distillation:

| | |
|---|---|
| 2,6-difluorochlorobenzene | 98.6 g |
| 2,3-difluorochlorobenzene | 68.9 g |
| 2,6-dichlorofluorobenzene | 20.3 g |
| 2,3-dichlorofluorobenzene | 20.4 g |
| 1,2,3-trifluorobenzene | 6.4 g |

Example 5 Reduction of 2,6-Dichlorofluorobenzene

A 100 mL round-bottomed flask equipped with a magnetic stirrer, thermometer and reflux condenser was charged with 3.3 g (0.02 mol) of 2,6-dichlorofluorobenzene, 2.56 g (0.04 mol) of ammonium formate and 0.2 g of 1 percent palladium on carbon in 36 mL of 2-propanol and 6 mL of water. The mixture was stirred at 55 ° C for 6 hr. The mixture contained 2,6-dichlorofluorobenzene (2,6-DCFB; 5 percent), o-chlorofluorobenzene (CFB; 80 percent) and fluorobenzene (FB; 15 percent).

Example 6 Reduction of 2,3-Dichlorofluorobenzene

A 100 mL round-bottomed flask equipped with a magnetic stirrer, thermometer and reflux condenser was charged with 3.3 g (0.02 mol) of 2,3-dichlorofluorobenzene, 2.52 g (0.04 mol) of ammonium formate and 0.2 g of 1 percent palladium on carbon in 36 mL of 2-propanol and 6 mL of water. The mixture was stirred at 45 ° C for 17 hr. The mixture contained 2,3-dichlorofluorobenzene (2,3-DCFB; 27.1 percent), CFB (51.7 percent) and FB (21.1 percent).

Example 7 Reduction of a Mixture of 2,3-Dichlorofluorobenzene and 2,6-Dichlorofluorobenzene

A mixture containing 84 percent of 2,3-DCFB/2,6-DCFB (5 g; 0.03 mol), sodium formate (2.25 g; 0.033 mol) and 0.5 g of 1 percent palladium on carbon in 75 mL of 2-propanol was heated at 70 ° C for 19 hr. The mixture contained DCFB (25 percent), CFB (52 percent) and FB (11 percent).

Example 8 Reduction of a Mixture of 2,3-Dichlorofluorobenzene and 2,6-Dichlorofluorobenzene

A 100 mL round-bottomed flask equipped with a magnetic stirrer, thermometer and reflux condenser was charged with 3.3 g (0.02 mol) of a 50/50 mixture of 2,3-DCFB and 2,6-DCFB, 2.52 g (0.04 mol) of ammonium formate and 0.2 g of 1 percent palladium on carbon in 40 mL of 2-propanol and 5 mL of water. The mixture was stirred at 50 ° C for 7 hr. The mixture contained 2,3-DCFB (31 percent), CFB (57 percent) and FB (12 percent). The mixture was separated by fractional distillation: 2,3-DCFB, b.p. 169-170 ° C; CFB, b.p. 138-140 ° C; and FB, b.p. 85-86 ° C.

## Claims

**1.** A process for preparing o-chlorofluorobenzene from 1,2,3-trichlorobenzene which comprises the following steps:

(a) exchanging one chlorine from 1,2,3-trichlorobenzene with fluorine to give a mixture of 2,6-dichlorofluorobenzene and 2,3-dichlorofluorobenzene;

(b) selectively reducing chlorine from the 2,6-dichlorofluorobenzene in admixture with 2,3-dichlorofluorobenzene by contacting the mixture with a hydrogen source in the presence of a palladium catalyst in an inert organic solvent at 50 to 70 ° C to give a mixture of fluorobenzene, o-chlorofluorobenzene and 2,3-dichlorofluorobenzene; and

(c) separating the components from the mixture and from each other.

European Patent Office

# EUROPEAN SEARCH REPORT

Application Number

EP 92 20 0866

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,P | JOURNAL OF FLUORINE CHEMISTRY. vol. 53, no. 3, August 1991, LAUSANNE CH pages 379 - 387; R. GARTH PEWS ET AL.: 'aromatic fluorine chemistry. part 5. preparation of 2,6-difluoroaniline and 1,2-difluorobenzene' * the whole document * | 1 | C07C17/20<br>C07C17/00<br>C07C25/13 |
| A<br><br>D | WO-A-9 011 987 (MALLINCKRODT INC.) * page 6 - page 7 *<br>& US-A-4 918 251 | 1 | |
| A<br><br>D | EP-A-0 371 563 (THE DOW CHEMICAL COMPANY) * page 5 - page 6; claim 1 *<br>& US-A-4 937 397 | 1 | |
| A,D | JOURNAL OF FLUORINE CHEMISTRY. vol. 2, no. 1, July 1972, LAUSANNE CH pages 19 - 26; R. H. SHILEY ET AL.: 'fluorination of 1,2,3-, 1,2,4- and 1,3,5-trihalobenzenes with potassium fluoride in dimethyl sulfone' * page 19 - page 22 * | 1 | TECHNICAL FIELDS SEARCHED (Int. Cl.5 ) |
| A | PATENT ABSTRACTS OF JAPAN vol. 12, no. 491 (C-554)21 December 1988 & JP-A-63 203 636 ( ASAHI GLASS CO LTD ) 23 August 1988 * abstract * | 1 | C07C |
| A,D | JOURNAL OF ORGANIC CHEMISTRY. vol. 42, no. 22, 28 October 1977, EASTON US pages 3491 - 3494; NICHOLAS A. CORTESE ET AL.: 'palladium-catalyzed reductions of halo- and nitroaromatic compounds with triethylammonium formate' | 1 | |

The present search report has been drawn up for all claims

| Place of search | Date of completion of the search | Examiner |
|---|---|---|
| BERLIN | 18 MAY 1992 | RUFET J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)